# EUROPEAN PATENT SPECIFICATION

(11) **EP 4 452 075 B1**
(45) Date of publication and mention of the grant of the patent: **23.07.2025**
(21) Application number: 22836183.8
(22) Date of filing: 16.12.2022
(51) Int. Cl.: A61B 6/03, A61B 6/42, A61B 6/04, A61B 6/00, G01T 1/20, G01T 1/29

(54) **PET IMAGING SYSTEM COOLING**
KÜHLUNG EINES HAUSTIERABBILDUNGSSYSTEMS
REFROIDISSEMENT DE SYSTÈME D'IMAGERIE PAR TEP

(30) Priority: 22.12.2021 US 202163292845 P; 01.03.2022 EP 22159335
(43) Date of publication of application: 30.10.2024
(73) Proprietor: Koninklijke Philips N.V., 5656 AG Eindhoven (NL); The Trustees of The University of Pennsylvania, Philadelphia, PA 19104 (US)
(72) Inventor: PERKINS, Amy, 5656 AG Eindhoven (NL); KARP, Joel, 5656 AG Eindhoven (NL); GEAGAN, Michael, 5656 AG Eindhoven (NL)
(74) Representative: Philips Intellectual Property & Standards
(86) International application number: PCT/EP2022/086265
(87) International publication number: WO 2023/117727

(56) References cited:
- CN-A- 106 901 772
- CN-A- 109 820 530
- CN-A- 110 897 649
- CN-U- 209 018 763
- JP-A- 2008 164 548
- US-A1- 2006 241 386
- US-A1- 2010 026 301
- US-A1- 2017 059 720
- US-A1- 2021 215 838

## Description

### TECHNICAL FIELD

The present disclosure relates to cooling in a positron emission tomography "PET" imaging system.

### BACKGROUND

PET imaging systems are used to study biological processes within the anatomy by means of a radiotracer. The radiotracer is attached to a molecule such as glucose or a ligand, and injected into the bloodstream of a subject. The bloodstream circulates the radiotracer within the anatomy, and the radiotracer is preferentially absorbed, or "taken up" in certain regions depending on the attachment molecule and the region's biological function. PET images representing the spatial distribution of the radiotracer within the anatomy are then generated from data generated by the PET imaging system. A clinician may study such images in order to make a diagnosis of the subject.

The spatial distribution of the radiotracer within the anatomy is determined by detecting gamma quanta that are emitted by the radiotracer whilst it decays. As the radiotracer decays, it emits positrons. The positrons are annihilated locally by electrons, resulting in the simultaneous emission of pairs of oppositely-directed gamma quanta. The emission of each pair of gamma quanta may be referred-to as a radioactive decay event, or simply an "event".

In order to detect the gamma quanta, a PET imaging system includes multiple gamma detectors. The gamma detectors are often grouped together in gamma detector modules, or simply "detector modules". The detector modules are arranged around a bore of the imaging system in order to detect the gamma quanta emitted from a subject within the bore. Each detector module includes a scintillator array that is coupled to a photodetector array. Each time a gamma quant is received by a scintillator array, a scintillation light pulse is generated in the scintillator array. The scintillation light pulse is then detected by its corresponding photodetector array. The positions at which each pair of gamma quanta associated with an event are detected, define a so-called "line of response" within the bore, and along which the event is deemed to have occurred. Image reconstruction techniques are used to reconstruct the lines of response recorded during an imaging session into PET images that represent the distribution of the radiotracer within the anatomy. In so-called time-of-flight "TOF" PET imaging, a more accurate position of the actual decay event along the LOR may be determined based on a time difference between the times of detection of the gamma quanta in each coincident pair.

One criterion that is used to assign the gamma quanta detected by the detector modules to pairs, and thereby define each event, is to determine whether gamma quanta are detected within a predetermined time interval. The time interval is defined by the time taken for a gamma quant to travel across the width of the bore of the PET imaging system. This criterion may be applied based on the detection times of the gamma quanta. For example, a temporal window may be opened when a gamma quant is received by a detector, and if another gamma quant is detected by a different detector within the time interval, the two gamma quanta may be assigned to a pair. Alternatively, the detection times of each received gamma quant, often referred-to as "timestamps", may be recorded and compared to identify pairs of gamma quanta with timestamps that occur within the time interval.

In order to determine when each gamma quant is detected, an analysis is performed on the timing, and sometimes also the spatial distributions, of electrical signals generated by the photodetector arrays in the detector modules in response to the scintillation light pulses. A photodetector array generates electrical signals in response to each scintillation light pulse generated in its corresponding scintillator array when a gamma quant is received. The detection times of the received gamma quanta and/or the light distribution generated in the scintillator array in response to the received gamma quanta, are referred-to as radioactive decay event data, or simply, "event data".

One factor affecting the performance of a PET imaging system is the accuracy with which the detection times of the received gamma quanta are determined. This is also known as "timing resolution" of the PET imaging system. In general, the timing resolution can be improved by recording the detection time of each gamma quant based on the detection time of as few scintillation photons as possible in each scintillation light pulse. In this regard, it is desired to detect each gamma quant based on the detection time of the first scintillation photon in each scintillation light pulse.

Single scintillation photons may be detected using a low-noise photodetector array that is highly-sensitive to scintillation photons. In order to avoid false detection events, the amount of electrical noise generated by the photodetector array should be low in comparison to the electrical signals generated in response to scintillation photons. One technique for reducing the amount of noise generated by a photodetector array is to cool the photodetector array. By way of an example, the noise performance of photodetector arrays that include silicon photomultiplier "SiPM" detectors may be improved by cooling.

Conventional cooling systems in PET imaging systems often employ fluid cooling, and fans. However, such cooling systems may be inappropriate for achieving the lower temperatures that are sought in order to improve the noise performance of the photodetector arrays in PET imaging systems. Moreover, the heat load of conventional cooling systems is often dissipated within the scanner room in which PET imaging is performed. Increasing this heat load in the pursuit of a lower temperature at the photodetector arrays creates a secondary challenge in removing this heat from the scanner room. Consequently, there remains room to improve the cooling of PET imaging systems in order to further improve their performance.

A document CN 109820530 A discloses a cooling system of a PET system detector, and relates to the technical field of medical imaging device. The cooling system comprises a plurality of detector modules. Each detector module comprises a plurality of sub-modules side by side and further comprises a liquid cooler with an outlet and an inlet, a liquid inlet main pipe connected with the outlet of the liquid cooler, a liquid outlet main pipe connected with the inlet of the liquid cooler, a first-stage parallel-connection liquid inlet pipeline connected with the liquid inlet main pipe, and a first-stage parallel-connection liquid outlet pipeline connected with the liquid outlet main pipe. Each detector module comprises a cooling station connected with the first-stage parallel-connection liquid inlet pipeline and the first-stage parallel-connection liquid outlet pipeline. Each sub-module comprises a cooling sub-station. The cooling system further comprises miniature electric adjusting valves arranged on the cooling sub-stations, temperature sensors arranged in the sub-modules, and a controller electrically connected with the miniature electric adjusting valves and the temperature sensors. The SiPM temperatures in the sub-modules are stabilized, and the temperatures of all the sub-modules are uniform.

A document US2017/059720A1 discloses a PET detector assembly. The PET detector assembly includes a plate having a first side and an opposite second side, the plate being fabricated from a thermally conductive material. The PET detector assembly also includes multiple PET detector units coupled to the first side of the plate. The PET detector assembly further includes a readout electronics section coupled to the second side of the plate, wherein, during operation, the readout electronics section generates heat that is transferred to the plate. The plate comprises a heat pipe disposed within the plate and configured to extract the heat from the plate and to transfer the heat away from the plate.

### SUMMARY

According to one aspect of the present disclosure, a positron emission tomography, PET, imaging system, is provided. The PET imaging system includes: a bore for receiving a subject, and a plurality of detector modules. Each detector module comprises a scintillator array coupled to a photodetector array. The detector modules are configured to generate event data in response to received gamma quanta. The detector modules are arranged around an axis of the bore such that the detector modules generate the event data in response to gamma quanta received from within the bore. Each detector module further comprises a cooling unit. The cooling unit includes a cooling fluid inlet, a cooling fluid outlet, a heat transfer region, and a passageway arranged between the cooling fluid inlet and the cooling fluid outlet for transferring heat from the heat transfer region via a cooling fluid inputted into the cooling fluid inlet. The heat transfer region of each cooling unit is thermally coupled to the photodetector array of the corresponding detector module for cooling the photodetector array. The cooling units are configured to be fluidically coupled in parallel to at least one cooling fluid source.

Since each detector module in the PET imaging system includes a cooling unit that cools the photodetector array in the detector module, and since the cooling units are configured to be fluidically coupled in parallel to at least one cooling fluid source, the detector modules are cooled individually. This arrangement reduces temperature variations between the photodetector arrays in different detector modules. This arrangement offers improved performance over existing cooling techniques in which photodetector arrays are cooled in series by a common cooling unit, and in which a photodetector array in a relatively downstream detector module typically has a higher temperature than a photodetector array in a relatively upstream detector module. In turn, improved PET imaging performance may be achieved with the proposed arrangement because the photodetector arrays in the PET imaging system are able to detect gamma quanta with a similar timing resolution. By contrast, the performance of existing PET imaging systems in which multiple detector modules are cooled in series by a common cooling unit, are limited by the timing accuracy achieved by the warmest, i.e. the most downstream, of the photodetector arrays.

Further aspects, features, and advantages of the present disclosure will become apparent from the following description of examples, which is made with reference to the accompanying drawings.

### BRIEF DESCRIPTION OF THE DRAWINGS

Fig. 1 is a schematic diagram illustrating a first view of an example of a PET imaging system 100, in accordance with some aspects of the present disclosure.
Fig. 2 is a schematic diagram illustrating a second view of an example of a PET imaging system 100, in accordance with some aspects of the present disclosure.
Fig. 3 is a schematic diagram illustrating a first example of a detector module 130₁ including a cooling unit 130c₁, in accordance with some aspects of the present disclosure.
Fig. 4 is a schematic diagram illustrating a second example of a detector module 130₁ including a cooling unit 130c₁, in accordance with some aspects of the present disclosure.
Fig. 5 is a schematic diagram illustrating a third example of a detector module 130₁ including a cooling unit 130c₁, in accordance with some aspects of the present disclosure.
Fig. 6 is a schematic diagram illustrating an example of an arrangement of detector modules 130ᵢ around an axis 120 of the bore 110 in a ring 250‴, in accordance with some aspects of the present disclosure.

### DETAILED DESCRIPTION

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity.

In the following description, reference is made to a PET imaging system. It is to be appreciated that the PET imaging system may be any type of PET imaging system, including a time-of-flight "TOF" PET imaging system, and a non-TOF-PET imaging. Reference is also made herein to examples of PET imaging systems that include multiple detector modules. The detector modules are arranged around an axis of a bore of the PET imaging system such that the detector modules generate event data in response to gamma quanta received from within the bore. In this respect, it is to be appreciated that the detector modules may be supported directly or indirectly by a mounting frame, often known as a gantry. The detector modules may be arranged to provide an axial field of view with a desired length. In this respect, it is to be appreciated that the length of the axial field of view may be sufficient to image a portion of a subject, such as the heart, the brain, the lungs, or it may include a substantial portion, or even the entire length, of a human subject. Some examples of the present disclosure may therefore be used to provide so-called "total-body" PET imaging systems.

As mentioned above, the performance of a PET imaging system may be improved by reducing the temperature of its photodetector arrays. However, the conventional cooling systems in PET imaging systems may be inappropriate for achieving the lower temperatures that are sought in order to improve the noise performance of the photodetector arrays.

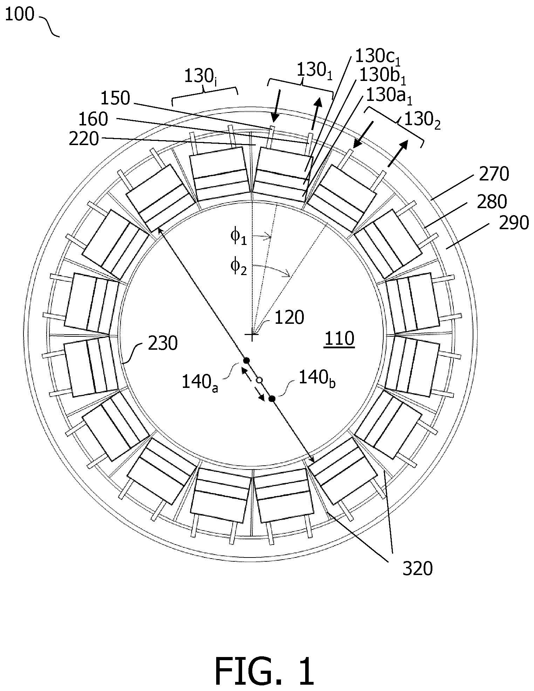

Fig. 1 is a schematic diagram illustrating a first view of an example of a PET imaging system 100, in accordance with some aspects of the present disclosure. The PET imaging system 100 includes a bore 110 for receiving a subject, and a plurality of detector modules 130_{1..i}. The bore 110 includes axis 120. In-use, the bore 110 may receive a subject, such as a human body, along the axis 120, by means of a patient bed (not illustrated in Fig. 1). The patient bed may be extended into the bore 110 in order to perform a PET imaging procedure on the subject. The PET imaging procedure may be used to detect a distribution of a radiotracer within a region of interest in the subject. A radiotracer such as ¹⁸F-fluoro-2-deoxy-D-glucose "FDG", or another radiotracer, may have been previously injected into the subject for this purpose. The region of interest may be a particular organ, such as the brain, the lungs, and so forth, or it may include a substantial portion of the body in a so-called "total-body" PET scan.

With continued reference to Fig. 1, each detector module 130_{1..i} includes a scintillator array 130a_{1..i} coupled to a photodetector array 130b_{1..i}. Each detector module is configured to generate event data in response to received gamma quanta, such as the gamma quanta 140ₐ, 140_{b} illustrated in Fig. 1. The event data represent detection times of the received gamma quanta 140ₐ, 140_{b} and/or a light distribution generated in the scintillator array in response to the received gamma quanta 140ₐ, 140_{b}. The scintillator array 130a_{1..i} may be formed from various scintillator materials that generate scintillation light in response to received gamma quanta. Scintillator materials such as lutetium yttrium orthosilicate "LYSO", bismuth germanate "BGO", and garnets such as gadolinium aluminum gallium garnet "GAGG", and so forth, are known for this purpose. The photodetector array 130b_{1..i} may be formed from various materials that are suitable for detecting the scintillation light. A silicon photodetector array that includes a plurality of silicon avalanche photodiodes may for example be used for this purpose. The photodetector array may include so called SiPM detectors. The photodetector array is optically coupled to the scintillator array such that a spatial distribution of the scintillation light that is generated in the scintillator array in response to received gamma quanta, is measured by the photodetector array. The photodetector array may be coupled to readout electronics for generating the event data from electrical signals generated by the photodetector array.

With continued reference to Fig. 1, the detector modules 130_{1..i} are arranged around the axis 120 of the bore 110 such that the detector modules generate the event data in response to gamma quanta received from within the bore. In this respect, the gamma detector modules 130_{1..i} may be arranged around the axis of the bore such that their radiation-receiving surfaces face the axis 120. The gamma detector modules 130_{1..i} may be arranged in the form of a ring, i.e. with the centers of their radiation-receiving faces disposed at a common radius from the axis 120 of the bore 110, and with their centers disposed at regular rotational angles ϕ₁ ϕ₂ etc. around the axis of the bore. In general, the PET imaging system may include one or more of such rings disposed along the axis 120. The gamma detector modules 130_{1..i} may also be supplied with a clock signal for use in determining the detection times of the received gamma quanta 140ₐ, 140_{b} with respect to a common reference time.

Each detector module 130_{1..i} illustrated in Fig. 1 also includes a cooling unit 130c_{1..i}. The cooling unit 130c_{1..i} includes a cooling fluid inlet 150, a cooling fluid outlet 160, a heat transfer region 170, and a passageway 180 arranged between the cooling fluid inlet and the cooling fluid outlet for transferring heat from the heat transfer region via a cooling fluid inputted into the cooling fluid inlet. The heat transfer region 170 of each cooling unit 130c_{1..i} is thermally coupled to the photodetector array 130b_{1..i} of the corresponding detector module for cooling the photodetector array. Moreover, the cooling units 130_{1..i} are configured to be fluidically coupled in parallel to at least one cooling fluid source. The cooling fluid source may for example be an open loop cooling system such as a mains water supply, or a single-pass water chiller coupled to a mains water supply. The cooling fluid source may alternatively be a re-circulating heat exchanger. The cooling fluid source may be located outside of a scanner room in which the PET imaging system is operated, thereby minimizing the heat load of the PET imaging system on the scanner room. The cooling fluid may for example include water. The cooling fluid may also include an antifreeze such as ethylene glycol, propylene glycol, and so forth. In some examples, the cooling fluid may provide a temperature of the photodetector array that is less than or equal to ten degrees centigrade, or less than or equal to five degrees centigrade. Such temperatures may permit the photodetector arrays to trigger the generation of timestamps based on a few photons, or even based on a single photon in the scintillation light pulses generated by received gamma quanta.

Since each detector module 130_{1..i} in the PET imaging system 100 includes a cooling unit 130_{1..i} that cools the photodetector array 130b_{1..i} in the detector module, and since the cooling units are configured to be fluidically coupled in parallel to at least one cooling fluid source, the detector modules are cooled individually. This arrangement reduces temperature variations between the photodetector arrays in different detector modules. This arrangement offers improved performance over existing cooling techniques in which photodetector arrays are cooled in series by a common cooling unit, and in which a photodetector array in a relatively downstream detector module typically has a higher temperature than a photodetector array in a relatively upstream detector module. In turn, improved PET imaging performance may be achieved with the proposed arrangement because the photodetector arrays in the PET imaging system are able to detect gamma quanta with a similar timing resolution. By contrast, the performance of existing PET imaging systems in which multiple detector modules are cooled in series by a common cooling unit, are limited by the timing accuracy achieved by the warmest, i.e. the most downstream, of the photodetector arrays.

The PET imaging system 100 described above may also include one or more additional features. These are described below with reference to further examples. It is noted that whilst the examples may be described individually, these examples may also be combined to provide further advantageous effects.

In one example, the cooling units 130c_{1..i} are located on trajectories extending radially outwards from the corresponding detector modules 130_{1..i} with respect to the axis 120 of the bore 110. This is illustrated in Fig. 1 for detector modules 130₁ and 130₂, which include such trajectories at the rotational anglesϕ₁ and ϕ₂. In contrast to existing cooling systems in which photodetector arrays are cooled using cooling plates disposed at the axial ends of the PET imaging system bore, locating the cooling units 130c_{1..i} on trajectories extending radially outwards from the corresponding detector modules 130_{1..i} permits the axial length of the bore to be extended by arranging the detector modules in multiple rings of detector modules along the axis of the bore, without the need to dispose cooling plates between the rings, and which might otherwise separate the rings, degrading the spatial resolution of the PET imaging system. Further advantages of locating the cooling units 130_{1..i} on trajectories that extend radially outwards from the corresponding detector modules 130_{1..i}, include improved access to the cooling units, and improved uniformity of the temperature within an individual photodetector array. In turn, this improves the performance of the PET imaging system because it reduces the variation in timing resolution along the axial direction. By contrast, existing cooling systems which cool photodetector arrays using cooling plates that are disposed at the axial ends of the PET imaging system bore, may suffer from temperature variations in an axial direction. For example, in existing cooling systems, the temperature may be higher in the center of the photodetector array than at the ends of the array, at which positions the heat is removed. This issue is exacerbated as the axial length of the photodetector array is increased. By instead locating the cooling units 130c_{1..i} on trajectories that extend radially outwards from the corresponding detector modules 130_{1..i}, cooling is effected by transferring heat from the photodetector array in a radial direction, and therefore a more uniform distribution of the temperature along the axial length of the photodetector array may be achieved.

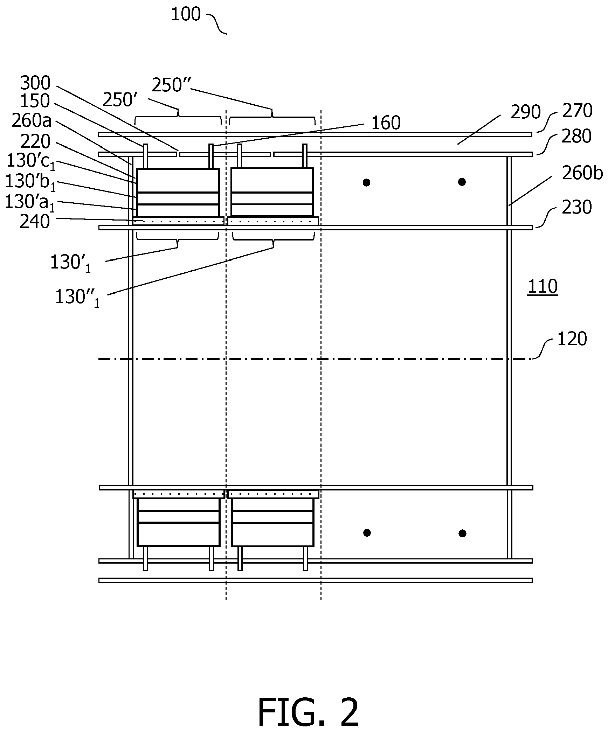

A further example of a PET imaging system in which the cooling units 130_{1..i} are located on trajectories extending radially outwards from the corresponding detector modules 130_{1..i} is illustrated in Fig. 2. Fig. 2 is a schematic diagram illustrating a second view of an example of a PET imaging system 100, in accordance with some aspects of the present disclosure. The second view illustrated in Fig. 2, represents an orthogonal view to the first view illustrated in Fig. 1. A further difference between Fig, 1 and Fig. 2 is that in Fig. 1 the detector modules 130_{1..i} are arranged around the axis 120 of the bore 110 in a single ring that is coaxial with the axis 120 of the bore 110, whereas in Fig. 2 the detector modules 130_{1..i} are arranged around the axis 120 of the bore 110 in multiple rings 250', 250", 250‴ that are coaxial with the axis 120 of the bore 110.

In one example, each detector module 130_{1..i} comprises a footprint defined by a portion of a surface of a cylinder occupied by a radiation receiving surface of the detector module, the cylinder being coaxial with the axis 120 of the bore 110, and wherein each cooling unit 130_{1..i} fits within the footprint of the corresponding detector module. This arrangement permits the axial length of the bore 110 to be extended by arranging the detector modules in multiple rings of detector modules along the axis of the bore, without the detector modules from neighboring rings obstructing one another. This example is illustrated in orthogonal views of Fig. 1 and Fig. 2, and wherein for instance in Fig. 1, the cylinder may identify with the tube 230 that extends into the plane of the drawing. By way of an example, in Fig. 1, the extent of the footprint for detector module 130₁ is bounded by the outer surface of the tube 230 between the sides of the radiation receiving face of detector module 130₁. In the orthogonal direction, and with reference to Fig. 2, the extent of the footprint for detector module 130'₁ is bounded by the outer surface of the tube 230 between the axial ends of the radiation-receiving surface of the detector module 130'₁.

In one example, the radiation receiving surface of each detector module 130_{1..i} occupies an angular range in a rotational direction f around the axis of the bore, and the radiation receiving surface of each detector module occupies a longitudinal extent along the axis 120 of the bore 110. Moreover, the cooling units 130c_{1..i} are arranged such that each cooling unit fits within the angular range, and within the longitudinal extent, occupied by the radiation receiving surface of the corresponding detector module 130_{1..i}. This arrangement also permits the axial length of the bore 110 to be extended by arranging the detector modules in multiple rings of detector modules along the axis of the bore, without the detector modules from neighboring rings obstructing one another. Moreover, it takes advantage of the increased space that is available in a rotational direction around the bore at positions that are further away from the bore axis. This example is illustrated in orthogonal views of Fig. 1 and Fig. 2, and wherein for instance in Fig. 1, the extent of the angular range in a rotational direction f for the detector module 130₁ is bounded by the sides of the radiation receiving face of detector module 130₁. In the orthogonal direction, and with reference to Fig. 2, the longitudinal extent occupied by the radiation receiving surface of the detector module 130₁ is bounded by the axial ends of the radiation-receiving surface of the detector module 130'₁.

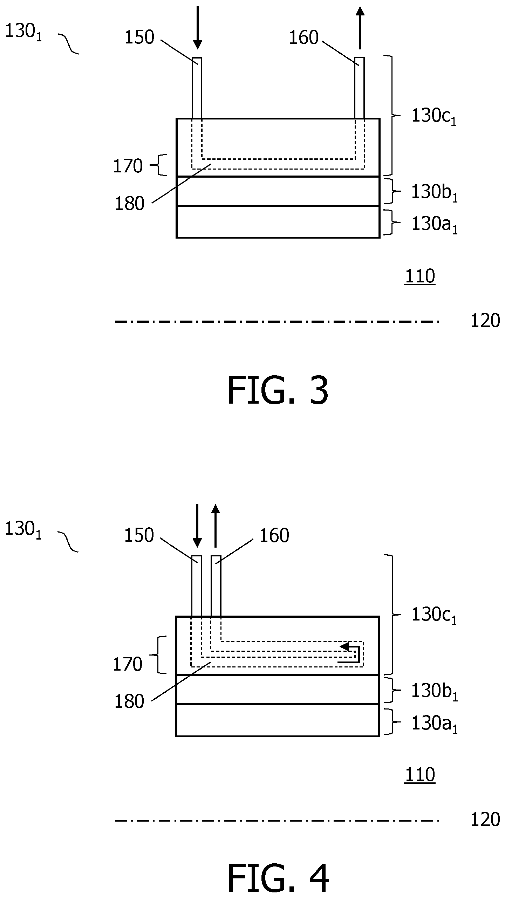

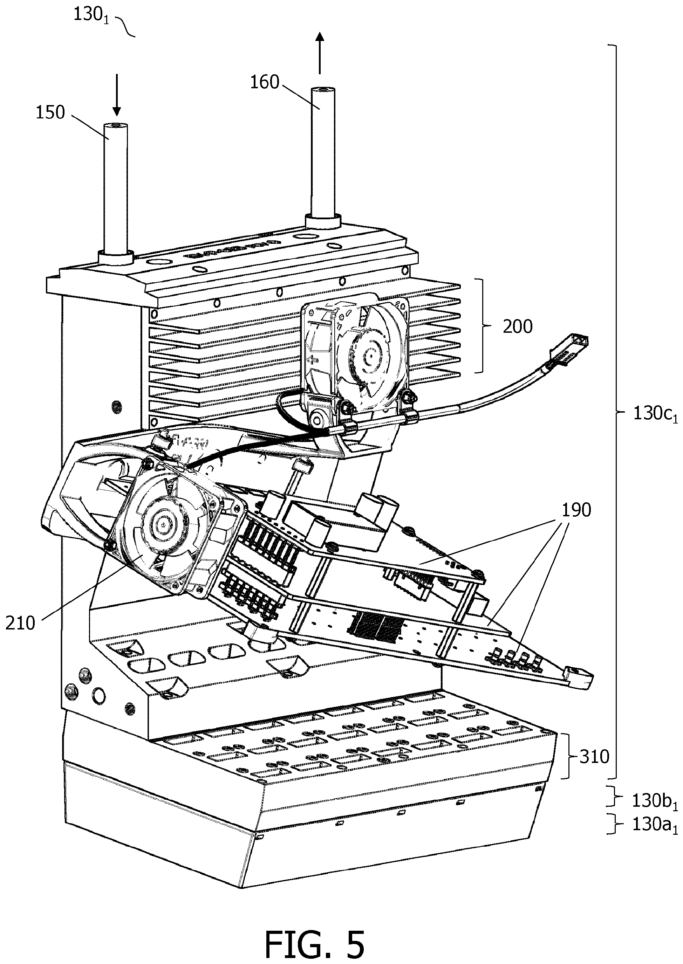

Fig. 3 is a schematic diagram illustrating a first example of a detector module 130₁ including a cooling unit 130c₁, in accordance with some aspects of the present disclosure. The cooling unit 130c₁ includes a cooling fluid inlet 150, a cooling fluid outlet 160, a heat transfer region 170, and a passageway 180 arranged between the cooling fluid inlet and the cooling fluid outlet for transferring heat from the heat transfer region via a cooling fluid inputted into the cooling fluid inlet. In the illustrated example, the heat transfer region 170 is a volumetric region. The volumetric region extends in an axial direction, i.e. along the bore axis 120, and in a radial direction with respect to the axis 120 of the bore 110, and also in a direction perpendicular to the plane of the illustration, i.e. in a transaxially direction. The heat transfer region 170 is in thermal communication with the passageway 180 such that cooling fluid passing through the passage way transfers heat away from the heat transfer region 170. The heat transfer region 170 of the cooling unit 130c₁ is thermally coupled to the photodetector array 130b₁ of the detector module 130₁ in order to cool the photodetector array. The heat transfer region 170 may be in direct or indirect contact with the photodetector array 130b₁. Thus, there may be heat-conductive material such as for example a thermally conductive paste, a thermally-conductive gasket, or a layer of metal, between the heat transfer region, and the photodetector array. In one example, a thermally conductive plate may be disposed between the heat transfer region 170 and the photodetector array 130b₁. Such a thermally conductive plate may be used to increase a thermal mass of the detector module 130c₁, or to provide a heat transfer region with a shape that matches a shape of the photodetector array 130b₁, as described later with reference to Fig. 5.

The cooling fluid inlet 150 and cooling fluid outlet 160 of the cooling unit 130c₁ may be formed at least in-part from a material such as a plastic, or a metal. The cooling unit may be formed at least in-part by a heat-conductive material. For example, part, or even all of the cooling unit may be formed from a metal such as aluminum, or copper. The passageway 180 may be formed within the cooling unit. For example, the passageway may be formed by drilling, or milling, one or more holes, or channels within the cooling unit. Alternatively, the passageway may be provided by one or more pipes within the cooling unit. The passageway may have various shapes. By way of some examples, the passageway 180 may have a u-shape as illustrated in Fig. 3, or it may have an alternating shape, such as a sinusoidal shape.

In operation, a hose (not illustrated) may be coupled to the cooling fluid inlet 150, in order to couple the cooling unit to at least one cooling fluid source. As mentioned above, the cooling fluid source may for example be an open loop cooling system. Examples of an open loop cooling system include a mains water supply, and a single-pass water chiller coupled to a mains water supply. In an open loop cooling system, the cooling fluid is not returned to the source, and thus the cooling fluid outlet 160 may be coupled via a hose to a waste water drainage system. The cooling fluid source may alternatively be a re-circulating heat exchanger. In this case, the cooling fluid inlets 150 and the cooling fluid outlets 160 of the cooling units 130c_{1..i} are configured to be fluidically coupled in parallel to the heat exchanger such that for each cooling unit 130c_{1..i}, a cooling fluid outputted by the heat exchanger is inputted to the fluid inlet 150 of the cooling unit and returned to the heat exchanger via the cooling fluid outlet 160 of the cooling unit before being inputted to the cooling fluid inlet 150 of another cooling unit.

In both the open loop cooling system, and in the re-circulating heat exchanger, the cooling units 130_{1..i} are configured to be fluidically coupled in parallel to the cooling fluid source. For instance, in the example of an open loop cooling system, the fluid inlets 150 of the cooling units in the PET imaging system may be fluidically coupled to a common manifold, and from which manifold a common source of cooling fluid is split into separate paths, each path being routed to one of the fluid inlets 150. In the example of a re-circulating heat exchanger, the fluid inlets 150 of the cooling units may likewise be fluidically coupled to a common manifold, and from which manifold a common source of cooling fluid is split into separate paths, each path being routed to one of the fluid inlets 150. The fluid outlets 160 may then be fluidically coupled together via a manifold such that their separate paths are re-combined in the manifold, and from which a common fluidic path is provided for the cooling fluid to return to the heat exchanger. The heat exchanger then cools the cooling fluid, and returns it to the cooling units. Alternatively, the fluid outlets 160 may be separately coupled to a common reservoir of the heat exchanger, where the returning cooling fluid is collected prior to being cooled, and then returned to the cooling units. The fluidic paths may be facilitated by hoses, pipes, ducts, and so forth.

In one example, each cooling unit 130c_{1..i} also includes a flow restrictor for generating a pressure gradient between the cooling fluid inlet 150 and the cooling fluid outlet 160 of the cooling unit for equalising a flow of the cooling fluid between the cooling units. By equalizing the flow of the cooling fluid, temperature variations between the photodetector arrays may be reduced. In so doing, the timing resolution of the PET imaging system may be improved. The flow restrictor may be provided by a narrowing in the aperture of the cooling fluid inlet 150, or in the passageway 180, or in the cooling fluid outlet 180, or in a hose that coupled to the cooling fluid inlet or outlet.

The inventors have recognized that when cooling the photodetector arrays of a PET imaging system in order to increase its performance, it becomes increasingly important to control the uniformity of the temperature within each photodetector array.

In one example, the passageway 180 of each cooling unit 130c_{1..i} is arranged such that a cooling fluid inputted to the cooling fluid inlet 150 of the cooling unit 130c_{1..i} flows in opposing directions within the heat transfer region before being outputted from the cooling fluid outlet 160 of the cooling unit. In this example, within the heat transfer region 170, at least a portion of the passageway extends in a direction parallel to the axis of the bore and returns in an opposing direction such that a cooling fluid inputted to the cooling fluid inlet 150 of the cooling unit 130_{1..i} flows in opposing directions within the heat transfer region before being outputted from the cooling fluid outlet 160 of the cooling unit.

In general, the temperature of the cooling fluid in the passageway tends to decrease when moving from a relatively upstream position to a relatively downstream position. In this example, the temperature gradients along the opposing paths of the passageway counteract one another to provide a more uniform average temperature along their length. In so doing, the heat transfer region 170 may be provided with a relatively uniform average temperature along the axis of the bore. This, in-turn, improves the timing performance of the detector modules, and consequently the performance of the PET imaging system. A high degree of uniformity in the average temperature may be obtained by arranging that the heat transfer region provides direct thermal contact between the opposing paths of the passageway continuously along their length. This example is illustrated in Fig. 4, which is a schematic diagram illustrating a second example of a detector module 130₁ including a cooling unit 130c₁, in accordance with some aspects of the present disclosure. In the example illustrated in Fig. 4, the passageway 180 extends in a direction parallel to the axis 120 of the bore 110, and then returns in an opposing direction. A high degree of uniformity in the average temperature may be obtained by arranging that within the heat transfer region, a separation between the opposing paths of the passageway is less than a separation between a closest of the opposing passageways and an interface between the cooling unit (130c_{1..i}) and the photodetector array (130b_{1..i}). Passageways with alternative shapes to that illustrated in Fig. 4 may also be used to provide the same effect. For example, the passageway may have multiple loops (not illustrated in Fig. 4). The opposing paths of the passageway 170 may be separated in a radial direction with respect to the axis of the bore, as illustrated in Fig. 3, or they may be separated in another direction, such as in a transaxial direction with respect to the axis of the bore. In one example, the opposing paths are separated in both a radial direction with respect to the axis of the bore, and also in a transaxial direction with respect to the axis of the bore. In the example illustrated in Fig. 5, plugs that identify the ends of these opposing paths are visible in the lower left-hand portion of the illustration.

In one example, each detector module includes a printed circuit board "PCB", and the cooling fluid passing through the passageway in the detector module is also used to cool the PCB. This example is illustrated in Fig. 5, which is a schematic diagram illustrating a third example of a detector module 130₁ including a cooling unit 130c₁, in accordance with some aspects of the present disclosure. In this example, each detector module further comprises at least one printed circuit board, PCB, 190. The at least one PCB comprises electronic processing circuitry. Each cooling unit further comprises one or more cooling fins 200 and a fan 210. Moreover, the one or more cooling fins 200 are thermally coupled to the cooling unit 130c_{1..i}, and the fan 210 is configured to transfer heat generated by the at least one PCB to the cooling fins for cooling the at least one PCB 190 via the cooling fluid inputted into the cooling fluid inlet of the cooling unit.

In so doing, in this example, the passageway 180 provides a common conduit for transferring heat, via the cooling fluid, away from both the PCB(s) and the photodetector array in the detector module. The fan may be configured to transfer heat generated by the at least one PCB to the cooling fins by arranging that an airflow generated by the fan passes over both the PCB(s) and the cooling fins. The PCB(s) may include processing circuitry for performing operations such as labelling the event data with the location at which gamma quanta were detected, timestamping the detected events, and so forth. Such processing circuitry may generate a significant amount of heat. The PCB(s) may also include power converter configured to supply power to the detector module. The power converter may likewise generate heat, and this heat may also be removed via the cooling fluid in the same manner. If the heat is not removed from the detector module, it risks that the temperature of the photodetector array increases, thereby degrading the performance of the PET imaging system.

In this example, the at least one PCB of each detector module 130_{1..i} may be mechanically coupled to the cooling unit 130c_{1..i} of the detector module, as illustrated in Fig. 5. Alternatively, the at least one PCB of each detector module 130_{1..i} may be coupled to a portion of the gantry. One or more further fans may also be provided, such as the fan that is mechanically coupled to the fins 200 in Fig. 5. An enclosure (not illustrated in Fig. 5) may also be provided that surrounds the PCB(s) and the fins and the PCB(s) such that air is re-circulated between the PCB and the fins within the enclosure, thereby improving the efficiency of the cooling.

As mentioned above, the detector modules 130₁ may also include a thermally conductive plate 310 disposed between the cooling unit 130c₁ and the photodetector array 130b₁. This is illustrated in the example detector module 130₁ in Fig. 5. The thermally conductive plate 310 may be used to provide a heat transfer region with a shape that matches a shape of the photodetector array 130b₁. The use of such a thermally conductive plate may also provide space in the detector module for the one or more PCBs 190. It may also facilitate a reduction in weight of the detector module 130₁. The thermally conductive plate 310 may be formed from various materials. For example, it may be formed from a metal such as aluminum, or copper.

The inventors have also recognized that as the photodetector arrays of a PET imaging system are cooled to lower temperatures, there are further challenges to be addressed in providing such temperatures, including the risk of condensation.

In one example, the PET imaging system 100 includes at least one humidity-controlled compartment 220. In this example, at least a portion of each detector module 130_{1..i}, and at least a portion of each corresponding cooling unit 130c_{1..i}, are arranged within the at least one humidity-controlled compartment.

The humidity-controlled compartment serves to prevent condensation from forming at key regions of the PET imaging system that are cooled by the cooling fluid. By way of an example, the scintillator arrays 130a_{1..i},, the photodetector arrays 130b_{1..i}, and at least a portion of the inlets 150 to the cooling units 130c₁, may be arranged within the at least one humidity-controlled compartment. One or more hoses that are coupled to the fluid inlets may also be arranged within the at least one humidity-controlled compartment. In so doing, condensation may be prevented from forming on these components. In this example, there may be a single humidity-controlled compartment for all detector modules, or there may be multiple humidity-controlled compartments. For example, each detector module 130ᵢ may be disposed within its own humidity-controlled compartment (as illustrated in Fig. 1), or a compartment may be provided for all of the detector modules in a ring. The humidity-controlled compartment may be configured to be coupled to a source of dry air, i.e. air having a dew point that is lower than the temperature of the cooling fluid. The humidity-controlled compartment may for example include an inlet such as a nozzle, or another type of fitting, and a hose, to enable the dry air to be supplied to the compartment. By way of some examples, the source of dry air may be a dehumidifier, such as a dessicant dehumidifier, or a refrigeration dehumidifier, a membrane dryer. Alternatively, the source of dry air may be a nitrogen generator that converts air to nitrogen.

In one example, an outer surface of a tube defines an inner surface of the at least one humidity-controlled compartment 220. In this example, the PET imaging system includes a tube 230. The tube is arranged coaxially with the axis 120 of the bore 110. The radiation receiving surfaces of the detector modules 130_{1..i} are arranged around an outer surface of the tube 230. Moreover, the outer surface of the tube defines an inner surface of the at least one humidity-controlled compartment 220. This example is illustrated with reference to Fig. 1 and in Fig. 2, and wherein a tube 230 is shown. The tube may define the bore 110 of the PET imaging system 100. The tube 230 may be formed from a material that is transparent to gamma quanta. Suitable materials include plastics such as Acrylonitrile Butadiene Styrene "ABS".

In one example, a thermal insulation layer 240 is provided, and the radiation receiving surfaces of the detector modules 130_{1..i} are separated from the outer surface of the tube by the thermal insulation layer 240. In so doing, it may be prevented that condensation forms on the cooled portion of the scintillator array. An example of such a thermal insulation layer 240 is illustrated in Fig. 2. Various materials may be used for the thermal insultation layer, including plastics such as polyurethane or polyethylene foam.

In one example, the detector modules are arranged in one or more rings, and a plate is disposed at each axial end of the one or more rings. In this example, the detector modules 130_{1..i} are arranged around the axis 120 of the bore 110 in one or more rings 250', 250", 250‴, the rings being coaxial with the axis 120 of the bore 110. The PET imaging system includes a plate 260a, 260b disposed at each axial end of the one or more rings; and a surface of each plate 260a, 260b provides an inner surface of the at least one humidity-controlled compartment 220.

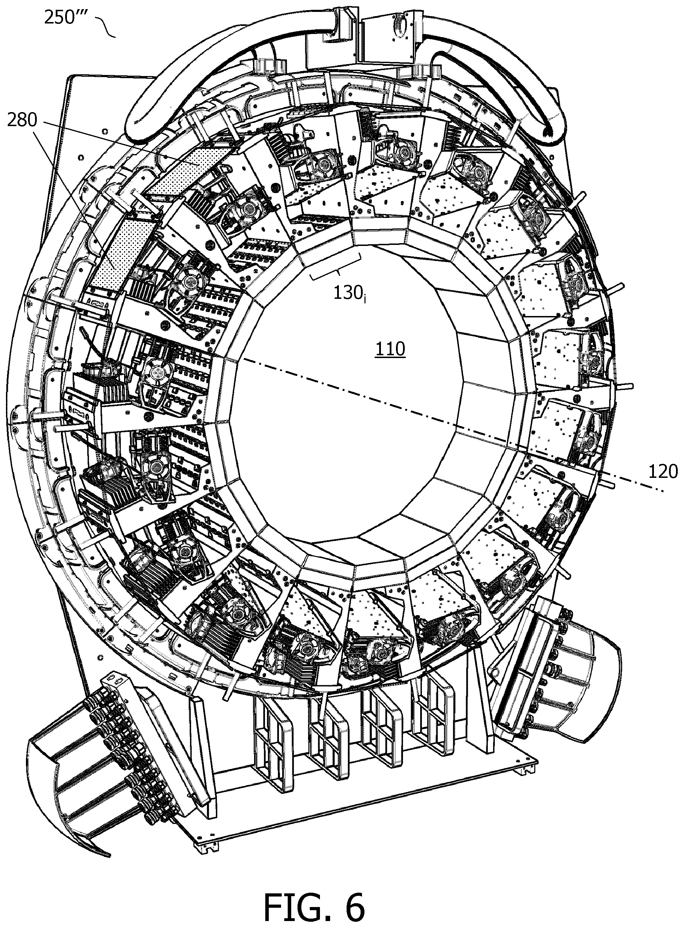

In this example, there may be a single ring with a plate is disposed at the axial ends of the ring, or there may be multiple rings, and a plate may be disposed at each end of the multiple rings. In other words, there may be two such plates in the PET imaging system. This example is illustrated with reference to Fig. 1, Fig. 2 and Fig. 6. In Fig. 2, there are multiple rings and there is one plate at each end, i.e. plates 260a and 260b. The plates 260a, 260b serve to define a common humidity-controlled compartment for the detector modules 130ᵢ. Such a configuration serves to homogenize the temperature of the photodetector arrays in the detector modules. The humidity-controlled compartment may also be sub-divided into multiple compartments. For example, dividing walls 320 may also be provided between the detector modules in a ring in order to provide multiple humidity-controlled compartments, as illustrated in Fig. 1. Such a configuration results in a common compartment for the detector modules at the same rotational angle ϕ₁, ϕ₂ around the ring, thereby homogenizing the temperature of the photodetector arrays in the detector modules at the same rotational angle ϕ₁, ϕ₂. A further illustration of this arrangement is provided in Fig. 6, which is a schematic diagram illustrating an example of an arrangement of detector modules 130ᵢ around an axis 120 of the bore 110 in a ring 250‴, in accordance with some aspects of the present disclosure. In Fig. 6, a portion of the cooling unit that extends radially outward from the bore serves to divide the detector modules 130ᵢ within a ring into separate humidity-controlled compartments. When there are multiple rings 250‴, the humidity-controlled compartments at the same rotational angle ϕ₁, ϕ₂ around the ring, may be common between the rings. In other words, dry air may be communicated in an axial direction between the detector modules that are at the same rotational angle ϕ₁, ϕ₂ around the ring. This arrangement likewise serves to homogenize the temperature of the photodetector arrays in the detector modules within each compartment. In particular it serves to homogenize the temperature axially along the bore 110.

In one example, the PET imaging system also includes an outer cover 270, and at least one baffle 280. The at least one baffle is disposed within the cover such that a radially innermost surface of the at least one baffle provides a radially outermost surface of the at least one humidity-controlled compartment 220, and such that an outer volume 290 is defined between the radially outermost surface of the at least one baffle and an inner surface of the cover. The fluid inlets 150 of the cooling units 130c_{1..i} are disposed within the outer volume 290. Moreover, the at least one humidity-controlled compartment 220 comprises at least one channel 300 configured to communicate air between the at least one humidity-controlled compartment 220 and the outer volume 290.

In so doing, it is provided that dry air leaks from the at least one humidity-controlled compartment 220 into the outer volume 290 via the at least one channel, thereby reducing the risk of condensation from forming on the fluid inlets 150 of the cooling units. This example is described with reference to Fig. 1, Fig. 2 and Fig. 6. The examples in Fig. 1 and Fig. 2 both include an outer cover 270 and a single baffle 280. Fig. 2 includes an example of a channel 300. In this example, a separate channel 300 is provided for each detector module 130'₁, and these are provided by way of one or more holes in the baffle. In general, the at least one channel may be provided by a hole, a pipe, or by gap. For example, a hole may be provided in the baffle(s), or a gap may be provided between the baffle(s) and a portion of the detector module(s). The dry air may be provided such that the dew point within the outer cover may for example be maintained at a temperature of minus ten degrees centigrade or lower, or minus twenty degrees centigrade or lower, for example. The dry air may be provided such that the relative humidity within the outer cover is less than five percent, for example.

The example in Fig. 6 includes multiple baffles 280, of which only two are shown. The outer cover is also omitted from the Fig. 6 example for ease of illustration. In the Fig. 6 example, each detector module 130ᵢ has its own baffle 280. The baffle may be removable for inspection purposes. A channel (not illustrated in Fig. 6 may be provided in each baffle. In general, the outer cover 270 and the baffle 280 may be provided from various materials. By way of some examples, various metals such as aluminum, steel, and so forth may be used. Alternatively, various plastics such as ABS, acrylic (e.g. Perspex^{®}), and polyethylene may be used.

The PET imaging system 100 may also be provided with a fan, or a pump, or a compressor. The fan, or the pump, or the compressor, is configured to supply dry air to the at least one humidity-controlled compartment 220 for controlling the humidity in the humidity-controlled compartment. The fan, or the pump, or the compressor, may be coupled to a dehumidifier, or a nitrogen generator for supplying the dry air, as mentioned above.

In one example, a detector module 130ᵢ for the PET imaging system 100 is provided. The detector module 130ᵢ comprises: a scintillator array 130aᵢ coupled to a photodetector array 130bᵢ; and a cooling unit 130cᵢ, the cooling unit including a cooling fluid inlet 150, a cooling fluid outlet 160, a heat transfer region 170, and a passageway 180 arranged between the cooling fluid inlet and the cooling fluid outlet for transferring heat from the heat transfer region via the cooling fluid; wherein the heat transfer region 170 of each cooling unit 130cᵢ is thermally coupled to the photodetector array 130bᵢ for cooling the photodetector array; and wherein the cooling unit 130cᵢ is arranged such that when the detector module 130ᵢ is arranged within the PET imaging system 100, the cooling unit is located on a trajectory extending radially outwards from the photodetector array 130bᵢ with respect to the axis 120 of the bore 110 of the PET imaging system.

The detector module 130ᵢ may also include one or more further features described above in relation to the detector module in the PET imaging system 100.

In one example, a cooling unit 130cᵢ for the PET imaging system 100 is provided. The cooling unit 130cᵢ comprises a cooling fluid inlet 150, a cooling fluid outlet 160, a heat transfer region 170, and a passageway 180 arranged between the cooling fluid inlet and the cooling fluid outlet for transferring heat from the heat transfer region via the cooling fluid. The cooling unit 130cᵢ is configured to be mechanically coupled to the detector module 130ᵢ such that when the detector module is arranged within the PET imaging system 100, the cooling unit 130cᵢ is located on a trajectory extending radially outwards from the photodetector array 130bᵢ with respect to the axis 120 of the bore 110 of the PET imaging system.

The cooling unit 130cᵢ may be configured to be mechanically coupled to the detector module 130ᵢ in various ways. For example, the cooling unit may include one or more holes for receiving or inserting bolt(s) in order to couple the cooling unit 130cᵢ to the detector module 130ᵢ. The cooling unit may alternatively include one or more fittings for mating with corresponding fitting(s) on the detector module 130ᵢ. The cooling unit may also include one or more further features described above in relation to the cooling unit in the PET imaging system 100.

The above examples are to be understood as illustrative of the present disclosure, and not restrictive. Further examples are also contemplated. For instance, features disclosed in relation to the detector module, or the cooling unit in the context of a PET imaging system, may also be incorporated into the detector module, or in the cooling unit as a standalone item. It is to be understood that a feature described in relation to any one example may be used alone, or in combination with other described features, and may be used in combination with one or more features of another of the examples, or a combination of other examples. Furthermore, modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A positron emission tomography, PET, imaging system (100), comprising:
a bore (110) for receiving a subject, the bore comprising an axis (120); and
a plurality of detector modules (130_{1..i});
wherein each detector module (130_{1..i}) comprises a scintillator array (130a_{1..i}) coupled to a photodetector array (130b_{1..i}), and wherein the detector module is configured to generate event data in response to received gamma quanta (140ₐ, 140_{b});
wherein the detector modules (130_{1..i}) are arranged around the axis (120) of the bore (110) such that the detector modules generate the event data in response to gamma quanta received from within the bore;
wherein each detector module (130_{1..i}) further comprises a cooling unit (130c_{1..i}), the cooling unit including a cooling fluid inlet (150), a cooling fluid outlet (160), a heat transfer region (170), and a passageway (180) arranged between the cooling fluid inlet and the cooling fluid outlet for transferring heat from the heat transfer region via a cooling fluid inputted into the cooling fluid inlet;
wherein the heat transfer region (170) of each cooling unit (130c_{1..i}) is thermally coupled to the photodetector array (130b_{1..i}) of the corresponding detector module for cooling the photodetector array; and
wherein the cooling units (130c_{1..i}) are configured to be fluidically coupled in parallel to at least one cooling fluid source; and
wherein within the heat transfer region (170), at least a portion of the passageway extends in a direction parallel to the axis of the bore and returns in an opposing direction such that a cooling fluid inputted to the cooling fluid inlet (150) of the cooling unit (130c_{1..i}) flows in opposing directions within the heat transfer region before being outputted from the cooling fluid outlet (160) of the cooling unit; and
wherein the at least a portion of the passageway comprises a first path and a second path, wherein the first path extends in the direction parallel to the axis of the bore and the second path returns in the opposing direction, and **characterised in that** the heat transfer region is configured to provide direct thermal contact between the opposing paths of the passageway continuously along their length.

2. The PET imaging system according to claim 1, wherein the at least a portion of the passageway comprises a first path and a second path, wherein the first path extends in the direction parallel to the axis of the bore and the second path returns in the opposing direction, and wherein the first path and the second path are separated in a radial direction with respect to the axis (120) of the bore (110).

3. The PET imaging system according to claim 1, wherein the cooling units (130c_{1..i}) are located on trajectories extending radially outwards from the corresponding detector modules (130_{1..i}) with respect to the axis (120) of the bore (110).

4. The PET imaging system according to claim 1 or claim 3, wherein each detector module (130_{1..i}) comprises a footprint defined by a portion of a surface of a cylinder occupied by a radiation receiving surface of the detector module, the cylinder being coaxial with the axis (120) of the bore (110), and wherein each cooling unit (130c_{1..i}) fits within the footprint of the corresponding detector module.

5. The PET imaging system according to claim 1 or claim 3, wherein the radiation receiving surface of each detector module (130_{1..i}) occupies an angular range in a rotational direction (ϕ) around the axis of the bore, and wherein the radiation receiving surface of each detector module occupies a longitudinal extent along the axis (120) of the bore (110); and
wherein the cooling units (130c_{1..i}) are arranged such that each cooling unit fits within the angular range, and within the longitudinal extent, occupied by the radiation receiving surface of the corresponding detector module (130_{1..i}).

6. The PET imaging system according to any one of claims 1 - 5, wherein each detector module further comprises at least one printed circuit board, PCB, (190);
wherein the at least one PCB comprises electronic processing circuitry; and
wherein each cooling unit further comprises one or more cooling fins (200) and a fan (210);
wherein the one or more cooling fins (200) are thermally coupled to the cooling unit (130c_{1..i}), and wherein the fan (210) is configured to transfer heat generated by the at least one PCB to the cooling fins for cooling the at least one PCB (190) via the cooling fluid inputted into the cooling fluid inlet of the cooling unit.

7. The PET imaging system according to any one of claims 1 - 6, wherein the at least one cooling fluid source comprises a re-circulating heat exchanger, and wherein the cooling fluid inlets (150) and the cooling fluid outlets (160) of the cooling units (130c_{1..i}) are configured to be fluidically coupled in parallel to the heat exchanger such that for each cooling unit (130c_{1..i}), a cooling fluid outputted by the heat exchanger is inputted to the fluid inlet (150) of the cooling unit and returned to the heat exchanger via the cooling fluid outlet (160) of the cooling unit before being inputted to the cooling fluid inlet (150) of another cooling unit.

8. The PET imaging system according to any one of claims 1 - 6, wherein the at least one cooling fluid source comprises an open loop cooling system.

9. The PET imaging system according to any one of claims 1 - 8, wherein each cooling unit (130c_{1..i}) includes a flow restrictor for generating a pressure gradient between the cooling fluid inlet (150) and the cooling fluid outlet (160) of the cooling unit for equalizing a flow of the cooling fluid between the cooling units.

10. The PET imaging system according to any one of claims 1 - 9, further comprising at least one humidity-controlled compartment (220); and
wherein at least a portion of each detector module (130_{1..i}), and at least a portion of each corresponding cooling unit (130c_{1..i}), are arranged within the at least one humidity-controlled compartment.

11. The PET imaging system according to claim 10, further comprising a tube (230);
wherein the tube is arranged coaxially with the axis (120) of the bore (110);
wherein the radiation receiving surfaces of the detector modules (130_{1..i}) are arranged around an outer surface of the tube (230); and
wherein the outer surface of the tube defines an inner surface of the at least one humidity-controlled compartment (220).

12. The PET imaging system according to claim 11, wherein the radiation receiving surfaces of the detector modules (130_{1..i}) are separated from the outer surface of the tube by a thermal insulation layer (240).

13. The PET imaging system according to any one of claims 10 - 12, wherein the detector modules (130_{1..i}) are arranged around the axis (120) of the bore (110) in one or more rings (250', 250", 250"'), the rings being coaxial with the axis (120) of the bore (110);
wherein the PET imaging system includes a plate (260a, 260b) disposed at each axial end of the one or more rings; and
wherein a surface of each plate (260a, 260b) provides an inner surface of the at least one humidity-controlled compartment (220).

14. The PET imaging system according to any one of claims 10 - 13, further comprising an outer cover (270), and at least one baffle (280);
wherein the at least one baffle is disposed within the cover such that a radially innermost surface of the at least one baffle provides a radially outermost surface of the at least one humidity-controlled compartment (220), and such that an outer volume (290) is defined between the radially outermost surface of the at least one baffle and an inner surface of the cover;
wherein the fluid inlets (150) of the cooling units (130c_{1..i}) are disposed within the outer volume (290); and
wherein the at least one humidity-controlled compartment (220) comprises at least one channel (300) configured to communicate air between the at least one humidity-controlled compartment (220) and the outer volume (290).

15. The PET imaging system according to any one of claims 10 - 14, further comprising a fan, or a pump, or a compressor, and wherein the fan, or the pump, or the compressor, is configured to supply dry air to the at least one humidity-controlled compartment (220) for controlling the humidity in the humidity-controlled compartment.

16. A detector module (130ᵢ) for a PET imaging system (100) comprising a bore (110) for receiving a subject, the bore comprising an axis (120), the detector module comprising:
a scintillator array (130aᵢ) coupled to a photodetector array (130bᵢ); and
a cooling unit (130cᵢ), the cooling unit including a cooling fluid inlet (150), a cooling fluid outlet (160), a heat transfer region (170), and a passageway (180) arranged between the cooling fluid inlet and the cooling fluid outlet for transferring heat from the heat transfer region via the cooling fluid;
wherein the heat transfer region (170) of each cooling unit (130cᵢ) is thermally coupled to the photodetector array (130bᵢ) for cooling the photodetector array; and
wherein the cooling unit (130cᵢ) is arranged such that when the detector module (130ᵢ) is arranged within the PET imaging system (100), the cooling unit is located on a trajectory extending radially outwards from the photodetector array (130bᵢ) with respect to the axis (120) of the bore (110) of the PET imaging system; and
wherein within the heat transfer region (170), at least a portion of the passageway extends in a direction parallel to the axis of the bore and returns in an opposing direction such that a cooling fluid inputted to the cooling fluid inlet (150) of the cooling unit (130c_{1..i}) flows in opposing directions within the heat transfer region before being outputted from the cooling fluid outlet (160) of the cooling unit; and
wherein the at least a portion of the passageway comprises a first path and a second path, wherein the first path extends in the direction parallel to the axis of the bore and the second path returns in the opposing direction, and **characterised in that** the heat transfer region is configured to provide direct thermal contact between the opposing paths of the passageway continuously along their length.

## Patentansprüche

1. Positronen-Emissions-Tomographie-, PET-, Bildgebungssystem (100), umfassend:
eine Bohrung (110) zum Aufnehmen eines Subjekts, wobei die Bohrung eine Achse (120) umfasst; und
eine Vielzahl von Detektormodulen (130_{1...i});
wobei jedes Detektormodul (130_{1...i}) eine Szintillatoranordnung (130a_{1..i}) umfasst, das mit einer Photodetektoranordnung (130b_{1..i}) gekoppelt ist, und wobei das Detektormodul konfiguriert ist, um als Reaktion auf empfangene Gammaquanten (140ₐ, 140_{b}) Ereignisdaten zu erzeugen;
wobei die Detektormodule (130_{1...i}) um die Achse (120) der Bohrung (110) herum angeordnet sind, sodass die Detektormodule die Ereignisdaten als Reaktion auf innerhalb der Bohrung empfangene Gammaquanten erzeugen;
wobei jedes Detektormodul (130_{1...i}) weiter eine Kühleinheit (130c_{1...i}) umfasst, die Kühleinheit einen Kühlfluideinlass (150), einen Kühlfluidauslass (160), einen Wärmeübertragungsbereich (170) und einen zwischen dem Kühlfluideinlass und dem Kühlfluidauslass angeordneten Durchgang (180) zum Übertragen von Wärme aus dem Wärmeübertragungsbereich über ein in den Kühlfluideinlass eingeführtes Kühlfluid beinhaltet;
wobei der Wärmeübertragungsbereich (170) einer jeden Kühleinheit (130c_{1..i}) zum Kühlen der Photodetektoranordnung thermisch mit der Photodetektoranordnung (130b_{1..i}) des entsprechenden Detektormoduls gekoppelt ist; und
wobei die Kühleinheiten (130c_{1..i}) konfiguriert sind, um fluidisch parallel mit mindestens einer Kühlfluidquelle gekoppelt zu sein; und
wobei sich innerhalb des Wärmeübertragungsbereichs (170) mindestens ein Abschnitt des Durchgangs in einer Richtung parallel zur Achse der Bohrung erstreckt, und in eine entgegengesetzte Richtung zurückführt, sodass ein in den Kühlfluideinlass (150) der Kühleinheit (130c_{1..i}) eingeführtes Kühlfluid innerhalb des Wärmeübertragungsbereichs in entgegengesetzte Richtungen fließt, bevor es aus dem Kühlfluidauslass (160) der Kühleinheit ausgegeben wird; und
wobei der mindestens eine Abschnitt des Durchgangs einen ersten Pfad und einen zweiten Pfad umfasst, wobei sich der erste Pfad in der Richtung parallel zur Achse der Bohrung erstreckt und der zweite Pfad in die entgegengesetzte Richtung zurückführt, und **dadurch gekennzeichnet, dass** der Wärmeübertragungsbereich konfiguriert ist, um einen direkten thermischen Kontakt zwischen den entgegengesetzten Pfaden des Durchgangs kontinuierlich über deren Länge hinweg bereitzustellen.

2. PET-Bildgebungssystem nach Anspruch 1, wobei der mindestens eine Abschnitt des Durchgangs einen ersten Pfad und einen zweiten Pfad umfasst, wobei sich der erste Pfad in der Richtung parallel zur Achse der Bohrung erstreckt und der zweite Pfad in die entgegengesetzte Richtung zurückführt, und wobei der erste Pfad und der zweite Pfad in radialer Richtung in Bezug auf die Achse (120) der Bohrung (110) getrennt sind.

3. PET-Bildgebungssystem nach Anspruch 1, wobei sich die Kühleinheiten (130c_{1..i}) auf Bewegungsbahnen befinden, die sich von den entsprechenden Detektormodulen (130_{1..i}) in Bezug auf die Achse (120) der Bohrung (110) radial nach außen erstrecken.

4. PET-Bildgebungssystem nach Anspruch 1 oder Anspruch 3, wobei jedes Detektormodul (130_{1..i}) eine Grundfläche umfasst, die durch einen Abschnitt einer Oberfläche eines Zylinders definiert ist, der von einer Strahlungsempfangsoberfläche des Detektormoduls eingenommen wird, wobei der Zylinder koaxial zur Achse (120) der Bohrung (110) liegt, und wobei jede Kühleinheit (130c_{1..i}) innerhalb der Grundfläche des entsprechenden Detektormoduls passt.

5. PET-Bildgebungssystem nach Anspruch 1 oder Anspruch 3, wobei die Strahlungsempfangsoberfläche jedes Detektormoduls (130_{1..i}) einen Winkelbereich in einer Drehrichtung (ϕ) um die Achse der Bohrung herum einnimmt und wobei die Strahlungsempfangsoberfläche jedes Detektormoduls eine Längserstreckung entlang der Achse (120) der Bohrung (110) einnimmt; und
wobei die Kühleinheiten (130c_{1..i}) angeordnet sind, sodass jede Kühleinheit innerhalb des Winkelbereichs und innerhalb der Längserstreckung passt, die von der Strahlungsempfangsoberfläche des entsprechenden Detektormoduls (130_{1..i}) eingenommen werden.

6. PET-Bildgebungssystem nach einem der Ansprüche 1-5, wobei jedes Detektormodul weiter mindestens eine Leiterplatte, PCB, (190) umfasst;
wobei die mindestens eine PCB eine elektronische Verarbeitungsschaltung umfasst; und
wobei jede Kühleinheit weiter eine oder mehrere Kühlrippen (200) und einen Lüfter (210) umfasst;
wobei die eine oder mehreren Kühlrippen (200) thermisch mit der Kühleinheit (130c_{1..i}) gekoppelt sind und wobei der Lüfter (210) konfiguriert ist, um von der mindestens einen PCB erzeugte Wärme über das in den Kühlflüssigkeitseinlass der Kühleinheit eingeführte Kühlfluid zum Kühlen der mindestens einen PCB (190) an die Kühlrippen zu übertragen.

7. PET-Bildgebungssystem nach einem der Ansprüche 1-6, wobei die mindestens eine Kühlfluidquelle einen Umwälzwärmetauscher umfasst und wobei die Kühlfluideinlässe (150) und die Kühlfluidauslässe (160) der Kühleinheiten (130c_{1..i}) konfiguriert sind, um fluidisch parallel mit dem Wärmetauscher gekoppelt zu sein, sodass für jede Kühleinheit (130c_{1..i}) ein vom Wärmetauscher ausgegebenes Kühlfluid in den Fluideinlass (150) der Kühleinheit eingeführt und über den Kühlfluidauslass (160) der Kühleinheit zum Wärmetauscher zurückgeführt wird, bevor es in den Kühlfluideinlass (150) einer anderen Kühleinheit eingeführt wird.

8. PET-Bildgebungssystem nach einem der Ansprüche 1-6, wobei die mindestens eine Kühlfluidquelle ein offenes Kühlkreislaufsystem umfasst.

9. PET-Bildgebungssystem nach einem der Ansprüche 1-8, wobei jede Kühleinheit (130c_{1..i}) einen Durchflussbegrenzer zum Erzeugen eines Druckgradienten zwischen dem Kühlfluideinlass (150) und dem Kühlfluidauslass (160) der Kühleinheit zum Ausgleichen eines Durchflusses des Kühlfluids zwischen den Kühleinheiten beinhaltet.

10. PET-Bildgebungssystem nach einem der Ansprüche 1-9, das weiter mindestens ein feuchtigkeitsgeregeltes Fach (220) umfasst; und
wobei mindestens ein Abschnitt eines jeden Detektormoduls (130_{1..i}) und mindestens ein Abschnitt einer jeden entsprechenden Kühleinheit (130c_{1..i}) innerhalb des mindestens einen feuchtigkeitsgeregelten Faches angeordnet sind.

11. PET-Bildgebungssystem nach Anspruch 10, das weiter ein Rohr (230) umfasst;
wobei das Rohr koaxial zur Achse (120) der Bohrung (110) angeordnet ist;
wobei die Strahlungsempfangsoberflächen der Detektormodule (130_{1..i}) um eine Außenoberfläche des Rohres (230) herum angeordnet sind; und
wobei die Außenoberfläche des Rohres eine Innenoberfläche des mindestens einen feuchtigkeitsgeregelten Faches (220) definiert.

12. PET-Bildgebungssystem nach Anspruch 11, wobei die Strahlungsempfangsoberflächen der Detektormodule (130_{1..i}) durch eine Wärmeisolationsschicht (240) von der Außenoberfläche des Rohres getrennt sind.

13. PET-Bildgebungssystem nach einem der Ansprüche 10 - 12, wobei die Detektormodule (130_{1..i}) in einem oder mehreren Ringen (250', 250", 250‴) um die Achse (120) der Bohrung (110) herum angeordnet sind, wobei die Ringe koaxial zur Achse (120) der Bohrung (110) sind;
wobei das PET-Bildgebungssystem eine Platte (260a, 260b) beinhaltet, die an jedem axialen Ende des einen oder der mehreren Ringe eingerichtet ist; und
wobei eine Oberfläche einer jeden Platte (260a, 260b) eine Innenoberfläche des mindestens einen feuchtigkeitsgeregelten Faches (220) bereitstellt.

14. PET-Bildgebungssystem nach einem der Ansprüche 10 - 13, das weiter eine äußere Abdeckung (270) und mindestens ein Leitblech (280) umfasst;
wobei das mindestens eine Leitblech innerhalb der Abdeckung eingerichtet ist, sodass eine radial innerste Oberfläche des mindestens einen Leitblechs eine radial äußerste Oberfläche des mindestens einen feuchtigkeitsgeregelten Faches (220) bereitstellt, und sodass zwischen der radial äußersten Oberfläche des mindestens einen Leitblechs und einer Innenoberfläche der Abdeckung ein Außenvolumen (290) definiert ist;
wobei die Fluideinlässe (150) der Kühleinheiten (130c_{1..i}) innerhalb des Außenvolumens (290) eingerichtet sind; und
wobei das mindestens eine feuchtigkeitsgeregelte Fach (220) mindestens einen Kanal (300) umfasst, der konfiguriert ist, um Luft zwischen dem mindestens einen feuchtigkeitsgeregelten Fach (220) und dem Außenvolumen (290) zu übertragen.

15. PET-Bildgebungssystem nach einem der Ansprüche 10 - 14, das weiter einen Lüfter oder eine Pumpe oder einen Kompressor umfasst, und wobei der Lüfter oder die Pumpe oder der Kompressor konfiguriert ist, um trockene Luft zu dem mindestens einen feuchtigkeitsgeregelten Fach (220) zum Regeln der Feuchtigkeit im feuchtigkeitsgeregelten Fach zuzuführen.

16. Detektormodul (130ᵢ) für ein PET-Bildgebungssystem (100), das eine Bohrung (110) zur Aufnahme eines Subjekts umfasst, wobei die Bohrung eine Achse (120) umfasst, wobei das Detektormodul umfasst:
eine Szintillatoranordnung (130aᵢ), die mit einer Photodetektoranordnung (130bᵢ) gekoppelt ist; und
eine Kühleinheit (130cᵢ), wobei die Kühleinheit einen Kühlfluideinlass (150), einen Kühlfluidauslass (160), einen Wärmeübertragungsbereich (170) und einen zwischen dem Kühlfluideinlass und dem Kühlfluidauslass angeordneten Durchgang (180) zum Übertragen von Wärme aus dem Wärmeübertragungsbereich über das Kühlfluid beinhaltet;
wobei der Wärmeübertragungsbereich (170) einer jeden Kühleinheit (130cᵢ) zum Kühlen der Photodetektoranordnung thermisch mit der Photodetektoranordnung (130bᵢ) gekoppelt ist; und
wobei die Kühleinheit (130cᵢ) angeordnet ist, sodass sich die Kühleinheit, wenn das Detektormodul (130ᵢ) innerhalb des PET-Bildgebungssystems (100) angeordnet ist, auf einer Bewegungsbahn befindet, die sich von der Photodetektoranordnung (130bᵢ) in Bezug auf die Achse (120) der Bohrung (110) des PET-Bildgebungssystems radial nach außen erstreckt; und
wobei sich innerhalb des Wärmeübertragungsbereichs (170) mindestens ein Abschnitt des Durchgangs in einer Richtung parallel zur Achse der Bohrung erstreckt, und in eine entgegengesetzte Richtung zurückführt, sodass ein in den Kühlfluideinlass (150) der Kühleinheit (130c_{1..i}) eingeführtes Kühlfluid innerhalb des Wärmeübertragungsbereichs in entgegengesetzte Richtungen fließt, bevor es aus dem Kühlfluidauslass (160) der Kühleinheit ausgegeben wird; und
wobei der mindestens eine Abschnitt des Durchgangs einen ersten Pfad und einen zweiten Pfad umfasst, wobei sich der erste Pfad in der Richtung parallel zur Achse der Bohrung erstreckt und der zweite Pfad in die entgegengesetzte Richtung zurückführt, und **dadurch gekennzeichnet, dass**
der Wärmeübertragungsbereich konfiguriert ist, um einen direkten thermischen Kontakt zwischen den entgegengesetzten Pfaden des Durchgangs kontinuierlich über deren Länge hinweg bereitzustellen.

## Revendications

1. Système d'imagerie par tomographie par émission de positrons, TEP, (100) comprenant :
un alésage (110) destiné à recevoir un sujet, l'alésage comprenant un axe (120) ; et
une pluralité de modules détecteurs (130_{1..i}) ;
dans lequel chaque module détecteur (130_{1..i}) comprend un réseau de scintillateurs (130a_{1..i}) couplé à un réseau de photodétecteurs (130b_{1..i}), et dans lequel le module détecteur est configuré pour générer des données d'événement en réponse aux quanta de gamma reçus (140ₐ, 140_{b}) ;
dans lequel les modules détecteurs (130_{1..i}) sont agencés autour de l'axe (120) de l'alésage (110) de sorte que les modules détecteurs génèrent les données d'événement en réponse aux quanta de gamma reçus de l'intérieur de l'alésage ;
dans lequel chaque module détecteur (130_{1..i}) comprend en outre une unité de refroidissement (130c_{1..i}), l'unité de refroidissement incluant une entrée de fluide de refroidissement (150), une sortie de fluide de refroidissement (160), une région de transfert de chaleur (170) et un passage (180) agencé entre l'entrée de fluide de refroidissement et la sortie de fluide de refroidissement pour transférer la chaleur de la région de transfert de chaleur par le biais d'un fluide de refroidissement introduit dans l'entrée de fluide de refroidissement ;
dans lequel la région de transfert de chaleur (170) de chaque unité de refroidissement (130c_{1..i}) est couplée thermiquement au réseau de photodétecteurs (130b_{1..i}) du module détecteur correspondant pour refroidir le réseau de photodétecteurs ; et
dans lequel les unités de refroidissement (130c_{1..i}) sont configurées pour être couplées fluidiquement en parallèle d'au moins une source de fluide de refroidissement ; et
dans lequel dans la région de transfert de chaleur (170), au moins une partie du passage s'étend dans une direction parallèle à l'axe de l'alésage et revient dans une direction opposée de sorte qu'un fluide de refroidissement introduit dans l'entrée de fluide de refroidissement (150) de l'unité de refroidissement (130c_{1..i}) s'écoule dans des directions opposées dans la région de transfert de chaleur avant d'être évacué par la sortie de fluide de refroidissement (160) de l'unité de refroidissement ; et
dans lequel la au moins une partie du passage comprend un premier trajet et un second trajet, dans lequel le premier trajet s'étend dans la direction parallèle à l'axe de l'alésage et le second trajet revient dans la direction opposée, et **caractérisé en ce que** la région de transfert de chaleur est configurée pour fournir un contact thermique direct entre les trajets opposés du passage en continu sur leur longueur.

2. Système d'imagerie TEP selon la revendication 1, dans lequel la au moins une partie du passage comprend un premier trajet et un second trajet, dans lequel le premier trajet s'étend dans la direction parallèle à l'axe de l'alésage et le second trajet revient dans la direction opposée, et dans lequel le premier trajet et le second trajet sont séparés dans une direction radiale par rapport à l'axe (120) de l'alésage (110).

3. Système d'imagerie TEP selon la revendication 1, dans lequel les unités de refroidissement (130c_{1..i}) sont situées sur des trajectoires s'étendant radialement vers l'extérieur à partir des modules détecteurs (130_{1..i}) correspondants par rapport à l'axe (120) de l'alésage (110).

4. Système d'imagerie TEP selon la revendication 1 ou la revendication 3, dans lequel chaque module détecteur (130_{1..i}) comprend une empreinte définie par une partie d'une surface d'un cylindre occupée par une surface de réception de rayonnement du module détecteur, le cylindre étant coaxial avec l'axe (120) de l'alésage (110), et dans lequel chaque unité de refroidissement (130c_{1..i}) s'insère dans l'empreinte du module détecteur correspondant.

5. Système d'imagerie TEP selon la revendication 1 ou la revendication 3, dans lequel la surface de réception de rayonnement de chaque module détecteur (130_{1..i}) occupe une plage angulaire dans une direction de rotation (ϕ) autour de l'axe de l'alésage, et dans lequel la surface de réception de rayonnement de chaque module détecteur occupe une étendue longitudinale le long de l'axe (120) de l'alésage (110) ; et
dans lequel les unités de refroidissement (130c_{1..i}) sont agencées de sorte que chaque unité de refroidissement s'insère dans la plage angulaire et dans l'étendue longitudinale occupées par la surface de réception de rayonnement du module détecteur (130_{1..i}) correspondant.

6. Système d'imagerie TEP selon l'une quelconque des revendications 1-5, dans lequel chaque module détecteur comprend en outre au moins une carte de circuit imprimé, PCB, (190) ;
dans lequel la au moins une PCB comprend des circuits de traitement électronique ; et
dans lequel chaque unité de refroidissement comprend en outre une ou plusieurs ailettes de refroidissement (200) et un ventilateur (210) ;
dans lequel les une ou plusieurs ailettes de refroidissement (200) sont couplées thermiquement à l'unité de refroidissement (130c_{1..i}), et dans lequel le ventilateur (210) est configuré pour transférer la chaleur générée par la au moins une PCB aux ailettes de refroidissement pour refroidir la au moins une PCB (190) par le biais du fluide de refroidissement introduit dans l'entrée de fluide de refroidissement de l'unité de refroidissement.

7. Système d'imagerie TEP selon l'une quelconque des revendications 1-6, dans lequel la au moins une source de fluide de refroidissement comprend un échangeur de chaleur à recirculation, et dans lequel les entrées de fluide de refroidissement (150) et les sorties de fluide de refroidissement (160) des unités de refroidissement (130c_{1..i}) sont configurées pour être couplées fluidiquement en parallèle de l'échangeur de chaleur de sorte que pour chaque unité de refroidissement (130c_{1..i}), un fluide de refroidissement évacué par l'échangeur de chaleur soit introduit dans l'entrée de fluide (150) de l'unité de refroidissement et renvoyé à l'échangeur de chaleur par le biais de la sortie de fluide de refroidissement (160) de l'unité de refroidissement avant d'être introduit dans l'entrée de fluide de refroidissement (150) d'une autre unité de refroidissement.

8. Système d'imagerie TEP selon l'une quelconque des revendications 1-6, dans lequel la au moins une source de fluide de refroidissement comprend un système de refroidissement en boucle ouverte.

9. Système d'imagerie TEP selon l'une quelconque des revendications 1-8, dans lequel chaque unité de refroidissement (130c_{1..i}) inclut un limiteur de débit pour générer un gradient de pression entre l'entrée de fluide de refroidissement (150) et la sortie de fluide de refroidissement (160) de l'unité de refroidissement pour équilibrer un débit du fluide de refroidissement entre les unités de refroidissement.

10. Système d'imagerie TEP selon l'une quelconque des revendications 1-9, comprenant en outre au moins un compartiment à humidité contrôlée (220) ; et
dans lequel au moins une partie de chaque module détecteur (130_{1..i}) et au moins une partie de chaque unité de refroidissement (130c_{1..i}) correspondante sont agencées à l'intérieur du au moins un compartiment à humidité contrôlée.

11. Système d'imagerie TEP selon la revendication 10, comprenant en outre un tube (230) ;
dans lequel le tube est agencé coaxialement avec l'axe (120) de l'alésage (110) ;
dans lequel les surfaces de réception de rayonnement des modules détecteurs (130_{1..i}) sont agencées autour d'une surface extérieure du tube (230) ; et
dans lequel la surface extérieure du tube définit une surface intérieure du au moins un compartiment à humidité contrôlée (220).

12. Système d'imagerie TEP selon la revendication 11, dans lequel les surfaces de réception de rayonnement des modules détecteurs (130_{1..i}) sont séparées de la surface extérieure du tube par une couche d'isolation thermique (240).

13. Système d'imagerie TEP selon l'une quelconque des revendications 10-12, dans lequel les modules détecteurs (130_{1..i}) sont agencés autour de l'axe (120) de l'alésage (110) dans un ou plusieurs anneaux (250', 250", 250‴), les anneaux étant coaxiaux avec l'axe (120) de l'alésage (110) ;
dans lequel le système d'imagerie TEP inclut une plaque (260a, 260b) disposée à chaque extrémité axiale des un ou plusieurs anneaux ; et
dans lequel une surface de chaque plaque (260a, 260b) fournit une surface intérieure du au moins un compartiment à humidité contrôlée (220).

14. Système d'imagerie TEP selon l'une quelconque des revendications 10-13, comprenant en outre une enveloppe extérieure (270) et au moins un déflecteur (280) ;
dans lequel le au moins un déflecteur est disposé à l'intérieur de l'enveloppe de sorte qu'une surface radialement la plus intérieure du au moins un déflecteur fournisse une surface radialement la plus extérieure du au moins un compartiment à humidité contrôlée (220) et de sorte qu'un volume extérieur (290) soit défini entre la surface radialement la plus extérieure du au moins un déflecteur et une surface intérieure de l'enveloppe ;
dans lequel les entrées de fluide (150) des unités de refroidissement (130c_{1..i}) sont disposées à l'intérieur du volume extérieur (290) ; et
dans lequel le au moins un compartiment à humidité contrôlée (220) comprend au moins un canal (300) configuré pour communiquer l'air entre le au moins un compartiment à humidité contrôlée (220) et le volume extérieur (290).

15. Système d'imagerie TEP selon l'une quelconque des revendications 10-14, comprenant en outre un ventilateur ou une pompe ou un compresseur, et dans lequel le ventilateur ou la pompe ou le compresseur est configuré pour fournir de l'air sec au au moins un compartiment à humidité contrôlée (220) pour contrôler l'humidité dans le compartiment à humidité contrôlée.

16. Module détecteur (130ᵢ) pour un système d'imagerie TEP (100) comprenant un alésage (110) destiné à recevoir un sujet, l'alésage comprenant un axe (120), le module détecteur comprenant :
un réseau de scintillateurs (130aᵢ) couplé à un réseau de photodétecteurs (130b;) ; et
une unité de refroidissement (130cᵢ), l'unité de refroidissement incluant une entrée de fluide de refroidissement (150), une sortie de fluide de refroidissement (160), une région de transfert de chaleur (170) et un passage (180) agencé entre l'entrée de fluide de refroidissement et la sortie de fluide de refroidissement pour transférer la chaleur de la région de transfert de chaleur par le biais du fluide de refroidissement ;
dans lequel la région de transfert de chaleur (170) de chaque unité de refroidissement (130cᵢ) est couplée thermiquement au réseau de photodétecteurs (130bᵢ) pour refroidir le réseau de photodétecteurs ; et
dans lequel l'unité de refroidissement (130cᵢ) est agencée de sorte que lorsque le module détecteur (130ᵢ) est agencé à l'intérieur du système d'imagerie TEP (100), l'unité de refroidissement soit située sur une trajectoire s'étendant radialement vers l'extérieur à partir du réseau de photodétecteurs (130bᵢ) par rapport à l'axe (120) de l'alésage (110) du système d'imagerie TEP ; et
dans lequel dans la région de transfert de chaleur (170), au moins une partie du passage s'étend dans une direction parallèle à l'axe de l'alésage et revient dans une direction opposée de sorte qu'un fluide de refroidissement introduit dans l'entrée de fluide de refroidissement (150) de l'unité de refroidissement (130c_{1..i}) s'écoule dans des directions opposées dans la région de transfert de chaleur avant d'être évacué par la sortie de fluide de refroidissement (160) de l'unité de refroidissement ; et
dans lequel la au moins une partie du passage comprend un premier trajet et un second trajet, dans lequel le premier trajet s'étend dans la direction parallèle à l'axe de l'alésage et le second trajet revient dans la direction opposée, et **caractérisé en ce que**
la région de transfert de chaleur est configurée pour fournir un contact thermique direct entre les trajets opposés du passage en continu sur leur longueur.
